# EUROPEAN PATENT APPLICATION

(11) **EP 2 359 744 A1**
(43) Date of publication of application: **24.08.2011**
(21) Application number: 10153469.1
(22) Date of filing: 12.02.2010
(51) Int. Cl.: A61B 3/12, G02B 21/06, G02B 27/00

(54) **Radiation emitting and receiving apparatus**

(71) Applicant: University of Northumbria at Newcastle, Newcastle upon Tyne, NE1 8ST (GB)
(72) Inventor: Faith, Joseph Emmanuel, Newcastle upon Tyne, Tyne & Wear, NE6 5AJ (GB); Allen, Joseph Ignatius Henry, Newcastle upon Tyne, Tyne & Wear, NE20 9JS (GB); Byrne, Phillip Owen, Whickham, Tyne & Wear, NE16 4PQ (GB)
(74) Representative: Vinsome, Rex Martin

(57) **Abstract**

An illumination apparatus (2) for emitting and receiving light for use in an imaging apparatus is disclosed. The illumination apparatus comprises a light source (8) for emitting light for illuminating an object, and first optical means (6, 12, 14, 16, 18) for directing light received from the object to a detector (20), and including at least one first transmitting element (6, 12, 14, 16, 18) for transmitting light. The light source is located within the convex hull defined by light transmitting parts of the first optical means and parts of the detector which can receive light from the object via the first optical means.

## Description

The present invention relates to a radiation emitting and receiving apparatus for emitting and receiving electromagnetic radiation for use in an imaging apparatus. The invention relates particularly, but not exclusively, to such a radiation emitting and receiving apparatus for through-lens axial illumination of a target.

Many imaging applications would benefit from through-lens axial illumination of targets in order to ensure sufficient illumination of the target area. This is particularly the case when targets having restricted apertures are illuminated, for example in retinal imaging.

It is known to achieve such illumination by means of a light source displaced from the optical axis of the illumination system and partially reflecting surfaces and/or prisms to reflect an illumination light beam to cause it to travel along the optical axis, while allowing passage of reflected light returning from the target along the optical axis.

Such arrangements suffer from the disadvantage that they are bulky, are often highly complex and costly, and can result in image quality reduction because of light scattering by the components of the illumination system.

Preferred embodiments of the present invention seek to overcome one or more of the above disadvantages of the prior art.

According to the present invention, there is provided an illumination apparatus for emitting and receiving electromagnetic radiation for use in an imaging apparatus, the illumination apparatus comprising:
at least one first radiation emitting element for emitting electromagnetic radiation for illuminating an object;
and
first optical means for directing electromagnetic radiation received from said object to detector means, and including at least one first transmitting element for transmitting electromagnetic radiation;
wherein at least one said first radiation emitting element is located in use within the convex hull defined by electromagnetic radiation transmitting parts of said first optical means and parts of said detector means which can receive electromagnetic radiation from said object via said first optical means.

The present invention is based on the surprising observation that a radiation emitting element located adjacent to an optical axis of an illumination apparatus may not cause significant deterioration in the quality of an image formed on the focal plane of the imaging system incorporating the illumination apparatus. This is because the radiation emitting element can be arranged such that it is not focussed in the focal plane of the detector means. As a result, a more compact axial illumination apparatus can be provided, which can be of simpler construction than existing apparatus using off-axis light sources. It will be understood by persons skilled in the art that the term "convex hull" means the boundary of the smallest volume enclosing the radiation transmitting parts of the first optical means and those parts of the detector in use which can receive electromagnetic radiation from the object via the first optical means (i.e. those parts of the detector where an image is formed). It will also be appreciated that the term "electromagnetic radiation" encompasses light in the visible and non-visible parts of the electromagnetic spectrum.

The apparatus may further comprise second optical means for transmitting electromagnetic radiation from at least one said first radiation emitting element to said object, and may include at least one second transmitting element for transmitting electromagnetic radiation.

At least one said first radiation emitting element may be an electroluminescent device and/or comprise at least part of at least one first wave guide.

For example, the apparatus may use a light emitting diode or laser diode as an illumination source, or may be an end of an optical fibre directing light from a light source in a different position.

At least one said first radiation emitting element may be a fluorescent element adapted to emit radiation in a first wavelength range when illuminated by radiation in a second wavelength range.

This provides the advantage of enabling radiation within a desired first wavelength range to be generated by means of a radiation source providing radiation within a second wavelength range which may be easier to generate.

The apparatus may further comprise at least one second radiation emitting element for illuminating at least one said first radiation emitting element with radiation in said second wavelength range, wherein at least one said second radiation emitting element in use is located within the convex hull defined by electromagnetic radiation transmitting parts of said first optical means and parts of said detector means which can receive electromagnetic radiation from said object via said first optical means.

This provides the advantage of enabling a particularly compact device to be constructed.

The position of at least one said radiation emitting element may be adjustable in use relative to the object.

This provides the advantage of enabling the beam angle of radiation illuminating the object to be adjusted.

At least one said first radiation emitting element may be embedded within a said first and/or second radiation transmitting element.

This provides the advantage of enabling a compact and simplified way of moving the first and/or second radiation emitting element and the first radiation transmitting element at the same time, which enables simplified control of the beam angle of radiation illuminating the object.

The apparatus may further comprise polariser means for adjusting the state of polarisation of radiation passing therethrough.

This element may incorporate a quarter wave retarding plate whose physical properties may be varied electronically.

The polariser means may comprise at least one circular polariser.

The apparatus may further comprise at least one filter for restricting passage of radiation outside of a predetermined frequency band.

At least one said filter may be tuneable.

The apparatus may further comprise image intensifier means for increasing the intensity of radiation directed to the detector means, and/or scanning means for enabling a radiation beam directed onto the object to be scanned.

The apparatus may further comprise detector means for receiving electromagnetic radiation from said object via said first optical means.

The detector means may be adapted to produce an output signal representing an intensity of electromagnetic radiation received from said object via said first optical means and/or may further comprise at least part of at least one second wave guide.

According to another aspect of the present invention, there is provided a method of illuminating an object, the method comprising:
causing at least one first radiation emitting element to emit electromagnetic radiation to illuminate an object; and
causing first optical means to direct electromagnetic radiation received from said object to detector means, wherein said first optical means includes at least one first transmitting element for transmitting electromagnetic radiation;
wherein at least one said first radiation emitting element is located in use within the convex hull defined by electromagnetic radiation transmitting parts of said first optical means and parts of said detector means which can receive electromagnetic radiation from said object via said first optical means.

Preferred embodiments of the present invention will now be described, by way of example only and not in any limitative sense with reference to the accompanying drawings, in which:-
Figure 1 is a schematic cross sectional side view of an illumination apparatus of a first embodiment of the present invention;
Figure 2 is a schematic cross sectional side view of an illumination apparatus of a second embodiment of the present invention;
Figure 3 is a schematic cross sectional side view of an illumination apparatus of a third embodiment of the present invention;
Figure 4 is a schematic cross sectional side view of an illumination apparatus of a fourth embodiment of the present invention;
Figure 5 is a schematic cross sectional side view of an illumination apparatus of a fifth embodiment of the present invention;
Figure 6 is a schematic cross sectional side view of an illumination apparatus of a sixth embodiment of the present invention;
Figure 7 is a schematic cross sectional side view of an illumination apparatus of a seventh embodiment of the present invention;
Figure 8 is a schematic cross sectional side view of an illumination apparatus of an eighth embodiment of the present invention;
Figure 9 is a schematic cross sectional side view of an illumination apparatus of a ninth embodiment of the present invention;
Figure 10 is a schematic cross sectional side view of an illumination apparatus of a tenth embodiment of the present invention;
Figure 11 is a schematic cross sectional side view of an illumination apparatus of an eleventh embodiment of the present invention; and
Figure 12 is a schematic cross sectional side view of an illumination apparatus of a twelfth embodiment of the present invention.

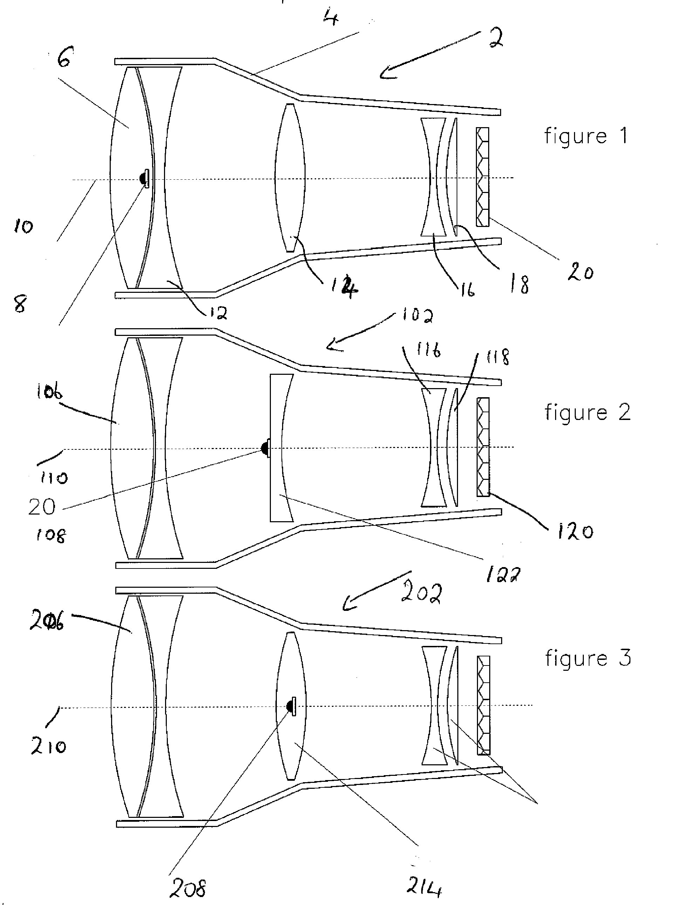

Referring to Figure 1, an electromagnetic radiation emitting and receiving apparatus 2 embodying the present invention includes a housing 4, and a first convex lens 6 for directing light from a light emitting diode (LED) 8 along an optical axis 10 of the apparatus onto an object (not shown), and receiving light reflected from the object. The LED 8 emits electromagnetic radiation in the range 200 nanometers to 50 micro meters, covering an operational band from the far UV to the far infrared. Electrical power is supplied to the LED by means of conducting transparent electrodes (not shown) formed from a number of materials which may be familiar to persons skilled in the art, such as indium tin oxide, in order to minimise the extent to which light passing through the apparatus 2 is obstructed.

The apparatus 2 also includes a first optical means for directing light reflected from the object onto an optical detector 20 such as a charge coupled device (CCD) array for producing a digital electrical output signal representing an image or information about the object, for example its spectral signature. The first optical means includes the first convex lens 6, a first concave lens 12, a second convex lens 14, a second concave lens 16, and a plano-convex lens 18. In this embodiment, the first convex lens 6 also forms second optical means for directing light from the light source 8 onto the object (not shown). The light source 8 is located within the convex hull defined by light transmitting components parts of components 6, 12, 14, 16 and 18, and that part of the detector which can receive light from the object (not shown) via the first optical means.

Figure 2 shows an arrangement of a second embodiment of the present invention, in which parts common to the embodiment of Figure 1 are denoted by like reference numerals but increased by 100, in which the second convex lens 14 of Figure 1 is replaced by a plano-concave lens 122, and light source 108 is mounted to the plano-concave 122 lens instead of being embedded within the first convex lens 6. Similarly, Figure 3 shows a third embodiment of the apparatus, in which parts common to the embodiment of Figure 1 are denoted by like reference numerals but increased by 200. The third embodiment differs from the arrangement of Figure 1 in that light source 208 is embedded in the second convex lens 214 instead of being embedded in the first convex lens 106 of Figure 1.

The operation of the embodiment of Figure 1 will now be described. It will be appreciated by persons skilled in the art that the embodiments of Figures 2 and 3 operate in a similar manner.

In order to carry out illumination of the object (not shown) along the optical axis 10, the LED 8 emits light along a cone centred about the optical axis 10 onto the object. Light reflected by the object is then directed by the first convex lens 6, the first concave lens 12, the second convex lens 14, second concave lens 16 and plano-convex lens 18 onto the detector 20 to form an image of the object. Because the axial positions of the lenses can be adjusted, by axial movement of the first convex lens 6, the light source 8 is moved to adjust the beam angle of the light illuminating the object. This provides a simplified and convenient way of adjusting the beam angle of the illumination light. It has been found that the location of the light source 8 on the optical axis does not cause significant deterioration of the quality of information received at the detector 20 because said the light source 8 is not focussed in the focal plane of the detector 20.

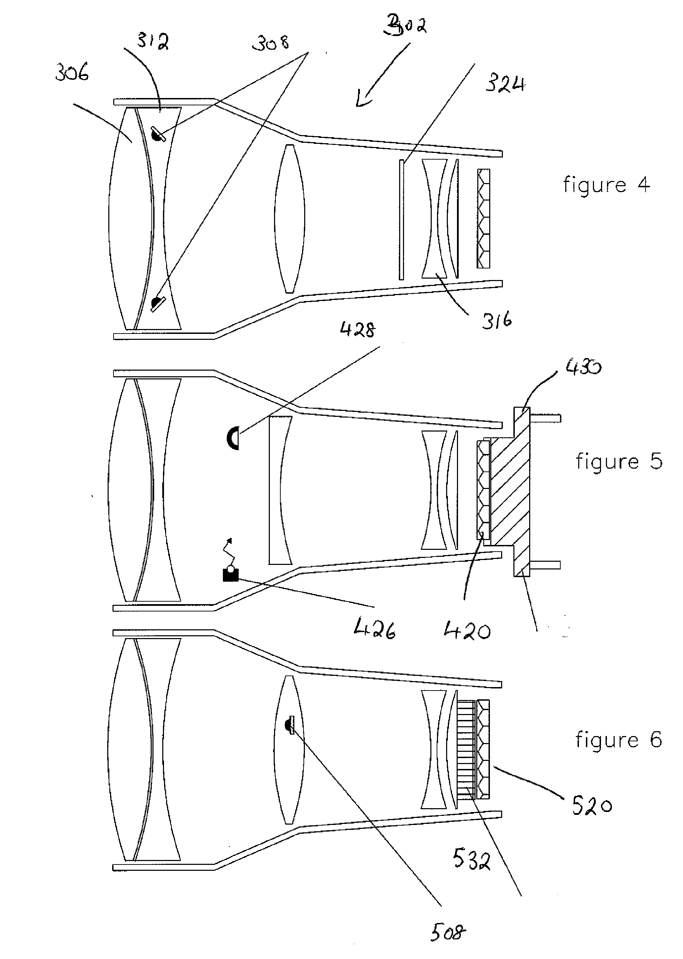

A fourth embodiment of the invention is shown in Figure 4, in which parts common to the embodiment of Figure 1 are denoted by like reference numerals but increased by 300. In the arrangement of Figure 4, a plurality of off-axis light sources 308 are arranged in a ring spaced from the optical axis, in order to further minimise image deterioration in the central part of the image, i.e. along the optical axis. The light sources 308 are embedded in the first concave lens 312, and an optical filter 324 is provided adjacent the second concave lens for selecting a desired wavelength range.

Figure 5 shows a fifth embodiment of the invention, in which parts common to the embodiment of Figure 2 are denoted by like reference numerals but increased by 300. The LED 108 of Figure 2 is replaced by a primary source 426 of electromagnetic radiation in a first, easily generated, wavelength range, and a fluorescent secondary source 428 for emitting radiation in a second, desired, wavelength range as a result of being excited by radiation from the first source 426. This provides the advantage of enabling emission of light in wavelength ranges in which it would be difficult to provide a compact primary source 426. The arrangement of Figure 5 also has a cooling device 430 thermally coupled to the detector 420 for cooling the detector.

Referring to Figure 6, in which parts common to the embodiment of Figure 3 are denoted by like reference numerals but increased by 300, a sixth embodiment of the invention is provided with an image intensifier 532 for intensifying the image received at the detector 520. The image intensifier 532 may be a channel plate multiplier, the operation of which will be familiar to persons skilled in the art. The light source 508 is located off-axis, in order to further minimise image deterioration in the central part of the image, i.e. along the optical axis.

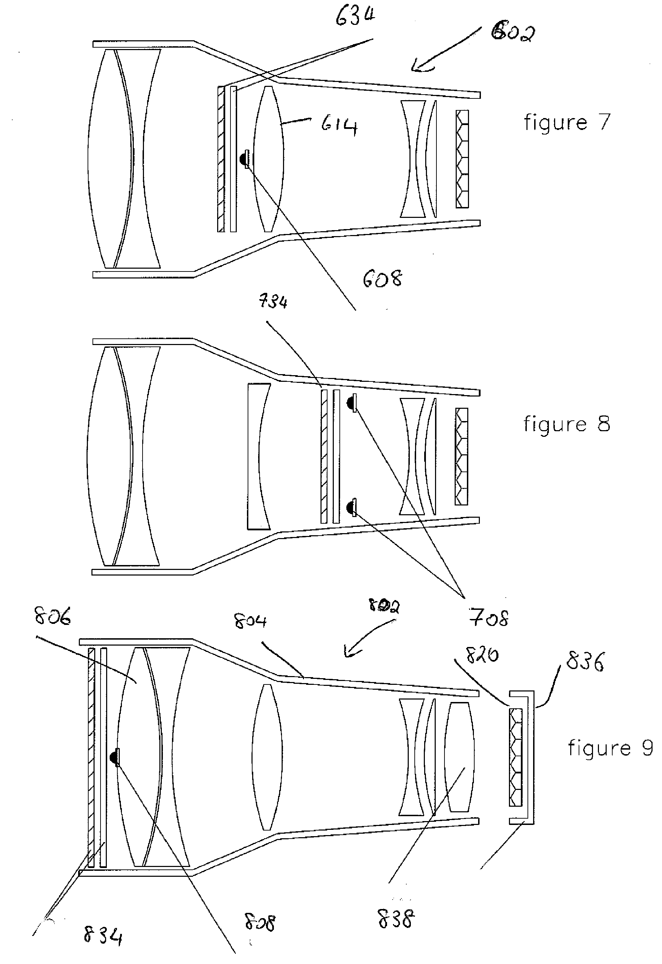

Figure 7 shows a seventh embodiment of the present invention, and parts common to the embodiment of Figure 1 are denoted by like reference numerals but increased by 600. The light source 608 is mounted between the second convex lens 614 and a circular polariser 634 comprising a quarter wave plate and a liner polariser for circularly polarising light directed from the light source 608 through the circular polariser 634 and lenses to the object. The polariser 634 adjusts the state of polarisation of light passing through it, for example to minimise light scattering from the optically transmissive components of the apparatus.

In the arrangement of Figure 8, in which parts common the to the embodiment of Figure 7 are denoted by like reference numerals but increased by 100, a plurality of off-axis light sources 708 are provided in an annular arrangement spaced from the optical axis and adjacent circular polariser 734 in order to minimise image deterioration in the region of the optical axis while maximising illumination of the object. In the arrangement of Figure 9, in which parts common to the embodiment of Figure 7 are denoted by like reference numerals but increased by 200, the light source 808 is mounted to the first convex lens 806 on a side facing the circular polariser 834. In addition, the detector 820 has a separate housing 836 and is separated from the main housing 804 of the apparatus 802 and a third convex lens 838 focuses light on the detector 820.

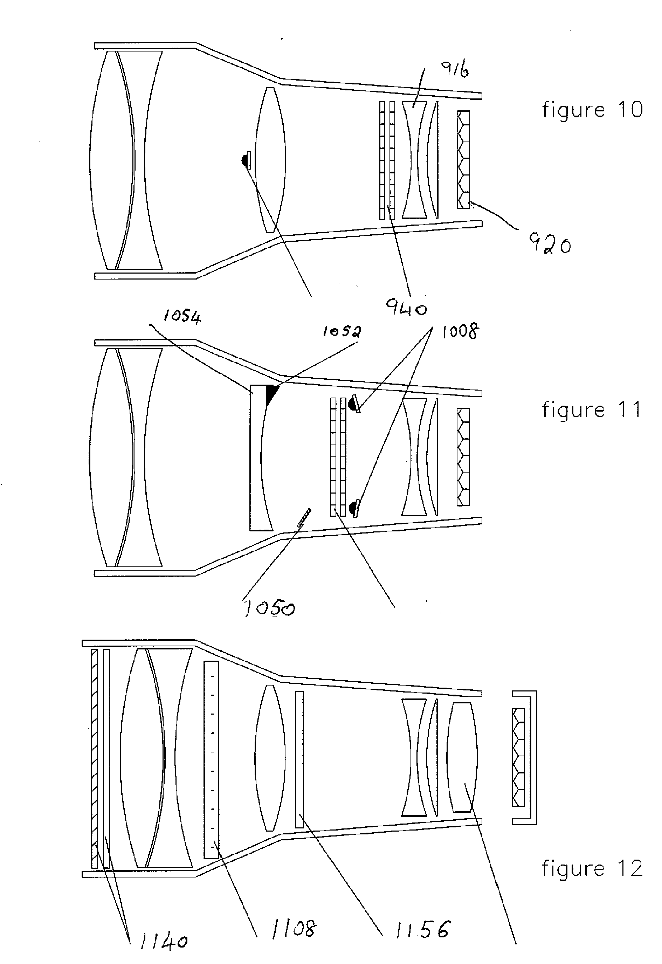

A tenth embodiment of the invention is shown in Figure 10, and parts common to the embodiment of Figure 7 are denoted by like reference numerals but increased by 300. The arrangement of Figure 10 differs from the embodiment of Figure 4 in that the circular polariser 634 is replaced by a tuneable optical filter 940, such as a Fabry-Perot etalon located adjacent the second concave lens 916, for selecting a narrow range of wave lengths which can be directed onto the detector 920.

Referring to Figure 11, an eleventh embodiment of the invention is provided with a moveable or steerable mirror 1050 placed within the convex hull. A pair of light sources 1008 are arranged to direct light towards an annular reflecting surface 1052 on plano-concave lens 1054, and the reflecting surface 1052 in turn directs the light towards the steerable mirror 1050 which can be moved via a computer signal or programme. This arrangement allows the target (not shown) to be scanned. This or a similar configuration allows line or spot scanning.

Figure 12 shows elements a circular polariser 1140 configured to generate circularly polarised light. A light source 1108 is a chemical flow cell used to generate electromagnetic radiation by means of chemical luminescence. Light received from the object then passes through an optical filter 1156.

It will be appreciated by person skilled in the art that the above embodiments have been described by way of example only, and not in any limitative sense, and that various alterations and modifications are possible without departure from the scope of the invention as defined by the appended claims. For example, although the specific embodiments described show a detector for receiving light reflected from the object and receiving light from the first optical means, it will be appreciated by persons skilled in the art that in certain circumstances the detector can be absent and the light from the first optical means be directly viewed by the eye.

## Claims

1. An illumination apparatus for emitting and receiving electromagnetic radiation for use in an imaging apparatus, the illumination apparatus comprising:
at least one first radiation emitting element for emitting electromagnetic radiation for illuminating an object;
and
first optical means for directing electromagnetic radiation received from said object to detector means, and including at least one first transmitting element for transmitting electromagnetic radiation;
wherein at least one said first radiation emitting element is located in use within the convex hull defined by electromagnetic radiation transmitting parts of said first optical means and parts of said detector means which can receive electromagnetic radiation from said object via said first optical means.

2. An apparatus according to claim 1, further comprising second optical means for transmitting electromagnetic radiation from at least one said first radiation emitting element to said object, and including at least one second transmitting element for transmitting electromagnetic radiation.

3. An apparatus according to claim 1 or 2, wherein at least one said first radiation emitting element comprises an electroluminescent device, and/or comprises at least part of at least one first wave guide.

4. An apparatus according to any one of the preceding claims, wherein at least one said first radiation emitting element comprises a fluorescent element adapted to emit radiation in a first wavelength range when illuminated by radiation in a second wavelength range.

5. An apparatus according to claim 4, further comprising at least one second radiation emitting element for illuminating at least one said first radiation emitting element with radiation in said second wavelength range, wherein at least one said second radiation emitting element in use is located within the convex hull defined by electromagnetic radiation transmitting parts of said first optical means and parts of said detector means which can receive electromagnetic radiation from said object via said first optical means.

6. An apparatus according to any one of the preceding claims, wherein the position of at least one said radiation emitting element in use is adjustable relative to the object.

7. An apparatus according to any one of the preceding claims, wherein at least one said first radiation emitting element is embedded within a said first and/or second transmitting element.

8. An apparatus according to any one of the preceding claims, further comprising polariser means for adjusting the state of polarisation of radiation passing therethrough.

9. An apparatus according to claim 8, wherein said polariser means comprises at least one circular polariser.

10. An apparatus according to any one of the preceding claims, further comprising at least one filter for restricting passage of radiation outside of a predetermined frequency band.

11. An apparatus according to claim 10, wherein at least one said filter is tuneable.

12. An apparatus according to any one of the preceding claims, further comprising image intensifier means for increasing the intensity of radiation directed to the detector means, and/or scanning means for enabling a radiation beam directed onto the object to be scanned.

13. An apparatus according to any one of the preceding claims, further comprising detector means for receiving electromagnetic radiation from said object via said first optical means.

14. An apparatus according to claim 13, wherein said detector means is adapted to produce an output signal representing an intensity of electromagnetic radiation received from said object via said first optical means and/or further comprises at least part of at least one second wave guide.

15. A method of illuminating an object, the method comprising:
causing at least one first radiation emitting element to emit electromagnetic radiation to illuminate an object; and
causing first optical means to direct electromagnetic radiation received from said object to detector means, wherein said first optical means includes at least one first transmitting element for transmitting electromagnetic radiation;
wherein at least one said first radiation emitting element is located in use within the convex hull defined by electromagnetic radiation transmitting parts of said first optical means and parts of said detector means which can receive electromagnetic radiation from said object via said first optical means.
